# EUROPEAN PATENT APPLICATION

(11) **EP 3 045 520 A1**
(43) Date of publication of application: **20.07.2016**
(21) Application number: 13893235.5
(22) Date of filing: 13.09.2013
(51) Int. Cl.: C12M 1/00, A61K 35/56, A61P 43/00, C12M 1/12

(54) **CELL SUSPENSION FILTRATION INSTRUMENT AND METHOD FOR PREPARING DRIP INFUSION SOLUTION CONTAINING CELL SUSPENSION**

(71) Applicant: Medinet Co., Ltd., Yokohama-shi, Kanagawa 222-0033 (JP)
(72) Inventor: NOGUCHI, Daisuke, Yokohama-shi Kanagawa 222-0033 (JP)
(74) Representative: Leeming, John Gerard
(86) International application number: PCT/JP2013/074809
(87) International publication number: WO 2015/037120

(57) **Abstract**

A filtration instrument to be used to remove foreign matter in a cell suspension wherein the filter container uses a transparent or semi-transparent material and is constructed so that solids that have been captured by the filter can be removed easily. Specifically, the filter container is constructed so that solids that have been captured by the filter can be removed easily by cutting the filter container by scissors. This construction serves to prevent the admixture of foreign matter in the cell suspension and, should foreign matter become admixed, facilitates the explanation of the cause of the admixture of foreign matter by removing and analyzing the foreign matter.

## Description

### TECHNICAL FIELD

The present invention relates to a filtration instrument to be used to remove foreign matter in a cell suspension and to a method for preparing a drip infusion solution containing a cell suspension using this instrument.

### BACKGROUND ART

Cell suspensions prepared by separating and washing cells from a cell suspension containing cultured cells and resuspending the separated target cells in physiological saline or the like are utilized as drip infusion solutions in immunotherapy and other therapeutic methods. Since the liquid components needed when culturing the cells and the liquid components when used in drip infusion or the like differ, a drip infusion solution containing a cell suspension is prepared by separating and washing the cells after cell culture, then resuspending them in physiological saline or the like.

Methods of separating the target cells and supernatant by centrifugation, passing the cell suspension through a filter member, capturing the target cells on the filter member, then recovering the target cells captured on the filter member by passing a liquid from the opposite direction to that in which the cell suspension was passed through the filter member, and the like are known as methods of separating and washing cells from a cell suspension.

In addition, it is necessary to prevent intermixing by foreign matter with the cell suspension when a cell suspension is used in therapy. For example, a disposable container for centrifugation of a shape capable of preventing the intermixing of bacteria and foreign matter and the like when separating and washing cells from a cell suspension containing cultured cells by centrifugation has been proposed (Patent Document 1).

### PRIOR ART DOCUMENTS

### PATENT DOCUMENTS

Japanese Laid-open Patent Application No. 2011-125813

### DISCLOSURE OF THE INVENTION

### PROBLEMS TO BE SOLVED BY THE INVENTION

Thus, various methods have been proposed for preventing intermixing by foreign matter during the step for separating and washing cells from a cell suspension containing cultured cells. Nonetheless, there is a step for injecting the cell suspension including the target cells into physiological saline in a drip infusion container after the step for separating and washing the target cells when preparing a drip infusion solution containing a cell suspension, and there is a risk of intermixing of foreign matter outside of the separation and washing step as well.

Should foreign matter become intermixed for whatever reason, it is necessary to analyze the foreign matter, clarify the cause of intermixing of foreign matter, and subsequently conduct cell culture and the like in addition to eliminating the cause of intermixing of foreign matter. Ante-factum measures to prevent intermixing by foreign matter have been studied in the prior art, but there has been no awareness of the importance of post-factum measures such as analyzing the foreign matter and clarifying the cause if foreign matter has intermixed for whatever reason.

### MEANS USED TO SOLVE THE ABOVE-MENTIONED PROBLEMS

The purpose of the present invention is to prevent intermixing by foreign matter or, should foreign matter become intermixed for any reason, analyze the foreign matter easily through the use of a specific filtration apparatus when separating and washing cells from a cell suspension containing cultured cells and preparing a drip infusion solution containing a cell suspension in which the target cells are resuspended.

Specifically, the invention according to a first aspect is a filtration instrument for filtering a cell suspension,
wherein the filtration instrument is characterized in comprising an inlet provided with a connection portion connectable with a container housing a cell suspension, an outlet provided with a connection portion connectable with cell suspension discharge means, a filter, and a filter container in which the filter is provided;
the filter container is of such configuration that the cell suspension does not contact the outside from the time of entering through the inlet, passing through the filter, and being discharged from the outlet;
the material of the filter container is a transparent or semitransparent material that allows solids captured by the filter to be confirmed visually; and
the filter container is of a construction that allows solids that have been captured by the filter to be removed easily.

In this filtration instrument, examples of a container housing a cell suspension include syringe barrel, culture bag, and the like.

In this filtration instrument, examples of cell suspension discharge means include an injection needle, pipette tip, and the like.

This filter container is of a construction that allows solids that have been captured by the filter to be removed easily. As a specific example, the filter container is constructed of a material that can be cut easily by scissors and constructed such that the solids captured by the filter can be removed easily by cutting by scissors.

In this filtration instrument, the inside diameter of the filter front chamber of the filter container is preferably larger than the inside diameter of the inlet and the outlet.

This filter container is also preferably of such configruation that the filter front chamber can be cut by scissors without cutting or damaging the filter.

The material of this filter container is not particularly restricted as long as it is a transparent or semitransparent material, can be cut easily by scissors, and is a material that does not damage cells even if it comes into contact with cells. Specific examples include polyvinyl chloride, polyester, polypropylene, polyethylene, polystyrene, polyamide, and other such resins.

The filter preferably has a pore size of 10-100 µm. The material of the filter is not particularly restricted as long as it is a material that does not damage cells. Examples include polyester, polyvinyl chloride, polypropylene, polyethylene, polystyrene, polyamide, polyimide, aramid, pulp, and the like.

The invention according to a second aspect is a method for preparing a drip infusion solution containing a cell suspension characterized in that
a cell suspension is prepared by separating a cell suspension containing cultured cells into a pellet containing cells and supernatant by centrifugation, removing the supernatant, and adding physiological saline to the pellet to achieve resuspension;
subsequently, the resuspended cell suspension is aspirated into a syringe barrel, an inlet of the filtration instrument according to Claims 1 to 4 is connected to the syringe barrel, and an injection needle is connected to the outlet of the filtration instrument; and
the injection needle is inserted into a drip infusion container housing physiological saline, and the cell suspension is injected into the drip infusion container by passing from the syringe barrel through the filtration instrument.

The invention according to a third aspect is a method for preparing an administration solution containing a cell suspension characterized in that an inlet of the filtration instrument of the invention according to the first aspect is connected to a container housing a cell suspension constituted of cells cultured ex vivo and a liquid that can be administered to a human body, and a cell suspension outlet is connected to the outlet of this filtration instrument; and
this cell suspension outlet is connected to an administration container housing a liquid that can be administered to a human body, and the cell suspension is injected into the administration container by passing from the container housing the cell suspension through the filtration instrument.

The liquid that can be administered to a human body is preferably physiological saline.

The administration solution is preferably one that can be administered as a drip infusion, or one that is injected intradermally, subcutaneously.

### EFFECT OF THE INVENTION

In the filtration instrument of the present invention, fresh intermixing of foreign matter can be prevented from the time the resuspended cell suspension is aspirated into the syringe barrel until it is injected into the drip infusion container since the cell suspension does not come into contact with the outside. Should foreign matter become intermixed in the resuspended cell suspension, the foreign matter is removed by the filter of the filter instrument, but the presence of this foreign matter can be easily recognized visually since the filtration container in the filtration instrument of the present invention is transparent or semitransparent. Moreover, the filtration instrument of the present invention allows the foreign matter captured by the filter to be removed easily by a method such as cutting the filter container by scissors or the like and allows the foreign matter to be analyzed easily. This facilitates explaining the cause of intermixing by foreign matter.

The filtration instrument of the present invention makes it possible to suppress cell damage due to excessive pressure exerted on the cells during cell suspension discharge by making the inside diameter of the filter front chamber of the filter container larger than the inside diameter of the inlet and the outlet.

### BRIEF DESCRIPTION OF THE DRAWINGS

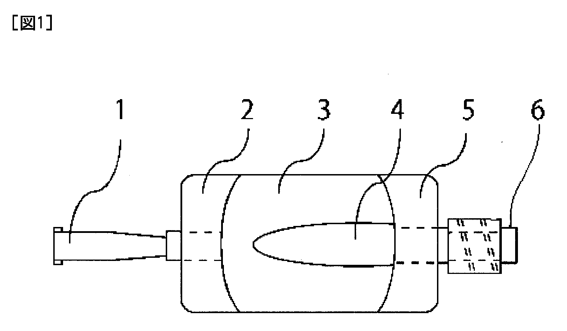

[Figure 1] Figure 1 is a diagram of an embodiment of a filtration instrument of the present invention.

### BEST MODE FOR CARRYING OUT THE INVENTION

The present invention is described below through examples, but various modifications can be allowed provided that no departure is made from the main point of the invention.

### Example 1

Figure 1 shows an explanatory diagram of a filtration instrument of this example. Polyvinyl chloride having a certain degree of strength was shaped, and an inlet 1 and outlet 6 were produced. Luer ports were provided to the inlet 1 and outlet 6 so that the syringe barrel and injection needle do not come loose during cell suspension discharge. Front chambers 2, 3, and 5 were produced using polyvinyl chloride. The polyvinyl chloride used in this example was soft enough to be cuttable by scissors and transparent enough that the interior of the front chamber could be checked visually. A filter 4 having an average pore size of 42 µm made of polyester was used as the filter 4. The filter 4 was positioned on the front chamber side of the outlet 6, and the filter 4 and outlet 6 were fixed by pressure exerted by a portion of front chamber 5. Meanwhile, the inlet 1 and the front chamber were fixed by pressure exerted by a portion of the front chamber 2. Both edges of front chamber 3 were curved to prevent liquid from remaining during cell suspension discharge. The diameter of the front chamber 3 was also set larger than the diameter of the inlet 1 and the outlet 6 so that excessive pressure would not be exerted during cell suspension discharge.

Next, the method for preparing a drip infusion solution containing a cell suspension using the filtration instrument of the present invention will be described.

The target cells were suspended in cell culture medium and, after culturing the cells by the usual method, the cell culture was separated into a pellet (cells) and supernatant by centrifugation. After aspirating off the supernatant, physiological saline was added to the centrifuge tube, and a cell suspension was prepared. The prepared cell suspension was aspirated into a syringe barrel.

The inlet 1 of the filtration instrument of the present invention and the aspiration port of the syringe barrel housing the cell suspension were connected, and the outlet 6 and a plastic needle were connected. Next, the plastic needle was inserted into a drip infusion container housing physiological saline, and the cell suspension was injected from the syringe barrel into the drip infusion container. This procedure filled the drip infusion with cell suspension that had been filtered by the filter 4 without contact with outside air.

After the drip infusion container was filled with cell suspension, a visual check was made that there was no residue in the front chamber 3 inside the filter container.

Additionally, the front chamber 3 was cut using stainless steel scissors, with care being taken not to cut the filter 4. A check was then made to ensure no foreign matter was present in the front chamber 3. Should foreign matter have been present, the foreign matter could have been removed and analyzed.

### INDUSTRIAL APPLICABILITY

The present invention makes it possible to prevent intermixing by foreign matter when preparing a drip infusion solution containing a cell suspension and, should foreign matter become intermixed, makes it possible to identify the presence of foreign matter easily and to analyze the foreign matter easily. This makes it possible to simultaneously prevent intermixing by foreign matter ante-factum and to take post-factum measures should foreign matter become intermixed, and to prepare a drip infusion solution containing a cell suspension simply and safely.

### DESCRIPTION OF THE NUMERICAL SYMBOLS

- 1:: Inlet
- 2:: Pressure-exerting portion of front chamber
- 3:: Front chamber
- 4:: Filter
- 5:: Pressure-exerting portion of front chamber
- 6:: Outlet

## Claims

1. A filtration instrument for filtering a cell suspension;
wherein said filtration instrument is **characterized in** comprising an inlet provided with a connection portion connectable with a container housing a cell suspension, an outlet provided with a connection portion connectable with cell suspension discharge means, a filter, and a filter container in which said filter is provided;
said filter container is of such configuration that the cell suspension does not contact the outside from the time of entering through the inlet, passing through the filter, and being discharged from the outlet;
the material of said filter container is a transparent or semitransparent material that allows solids captured by the filter to be confirmed visually; and
said filter container is of a construction that allows solids that have been captured by the filter to be removed easily.

2. The filtration instrument according to Claim 1 **characterized in that** said filter container is a structure allowing solids captured by the filter to be removed easily by being constructed of a material that can be cut easily by scissors.

3. The filtration instrument according to Claims 1 or 2 **characterized in that** the inside diameter of the filter front chamber of said filter container is larger than the inside diameter of the inlet and the outlet.

4. The filtration instrument according to Claims 1 to 3 **characterized in that** said filter container is of such configruation that the filter front chamber can be cut by scissors without cutting or damaging the filter.

5. A method for preparing a drip infusion solution containing a cell suspension, **characterized in that**:
a cell suspension is prepared by separating a cell suspension containing cultured cells into a pellet containing cells and supernatant by centrifugation, removing the supernatant, and adding physiological saline to the pellet to achieve resuspension;
subsequently, the resuspended cell suspension is aspirated into a syringe barrel, an inlet of the filtration instrument according to Claims 1 to 4 is connected to said syringe barrel, and an injection needle is connected to the outlet of said filtration instrument; and
said injection needle is inserted into a drip infusion container housing physiological saline, and said cell suspension is injected into the drip infusion container by passing from the syringe barrel through said filtration instrument.

6. A method for preparing an administration solution containing a cell suspension, **characterized in that** an inlet of the filtration instrument according to Claims 1 to 4 is connected to a container housing a cell suspension constituted of cells cultured ex vivo and a liquid that can be administered to a human body, and a cell suspension outlet is connected to the outlet of said filtration instrument; and
said cell suspension outlet is connected to an administration container housing a liquid that can be administered to a human body, and said cell suspension is injected into the administration container by passing from the container housing the cell suspension through the filtration instrument.
